# EUROPÄISCHE PATENTSCHRIFT

(11) **EP 0 617 918 B1**
(45) Veröffentlichungstag und Bekanntmachung des Hinweises auf die Patenterteilung: **12.12.2001**
(21) Anmeldenummer: 94101938.2
(22) Anmeldetag: 09.02.1994
(51) Int. Cl.: A61B 5/0452, A61B 5/0402, A61N 1/368, A61N 1/39

(54) **Vorrichtung zur Signalanalyse des elektrischen Potentialverlaufes der Herzerregung**
Apparatus for analysis of potential course signals of an electrocardiogram
Appareil pour l'analyse des signaux de l'écoulement électrique du potentiel de l'électrocardiogramme

(30) Priorität: 31.03.1993 DE 4310412
(43) Veröffentlichungstag der Anmeldung: 05.10.1994
(73) Patentinhaber: Zehender, Manfred, Dr., D-79110 Freiburg (DE)
(72) Erfinder: Zehender, Manfred, Dr., D-79110 Freiburg (DE)
(74) Vertreter: Patent- und Rechtsanwaltssozietät, Maucher, Börjes & Kollegen

(56) Entgegenhaltungen:
- EP-A- 0 554 208
- WO-A-92/16143
- DE-A- 3 818 136
- GB-A- 2 182 852

## Beschreibung

Die Erfindung betrifft eine Vorrichtung zur Signalanalyse bei einer Mangeldurchblutung am Herzen und/oder einer Herzrhythmusstörung mittels Meßelektroden zum Erfassen des elektrischen Potentialverlaufes der Herzerregung über die Zeit.

Der plötzliche Herztod stellt eine häufige Todesursache dar. Zahlreiche Untersuchungen haben gezeigt, daß der plötzliche Herztod in der überwiegenden Zahl der Fälle durch schnelle Herzrhythmusstörungen verursacht wird.

Aus der deutschen Zeitschrift für Kardiologie, 78,5,1989, Seiten 55 bis 62 ergeben sich Hinweise, aus denen geschlossen werden kann, daß eine lebensbedrohliche Herzrhythmusstörung in etwa einem Drittel bis der Hälfte der Fälle durch transitorische Mangeldurchblutung des Herzens - die sogenannte Myokardischämie - ausgelöst wird. Dabei geht eine solche Mangeldurchblutung der Herzrhythmusstörung in der Regel einige Minuten voraus und führt zu einer Instabilität der elektrischen Erregungsausbreitung am Herzmuskel mit der Gefahr der Auslösung der lebensbedrohlichen Herzrhythmusstörung. Dabei wird diese nur in relativ seltenen Fällen von dem Betroffenen selbst als Schmerzsymptomatik verspürt, die eventuell Anlaß zu Gegenmaßnahmen wie Unterbrechung einer körperlichen Tätigkeit, Einnahme von Medikamenten oder Aufsuchen eines Arztes sein könnte.

Aus DE-38 18 136 A1 ist es bekannt, zur Bekämpfung von Herzrhythmusstörungen implantierbare Defibrillationssysteme vorzusehen.

Dabei wird ein Defibrillator meist im Bauchraum implantiert, von welchem Defibrillationselektroden zum Herzen verlaufen. Diese werden jeweils im Bereich der rechten Herzkammer und des rechten Herzvorhofes bzw. dem Übergang zur oberen Hohlvene plaziert. Gegebenenfalls können zusätzlich Flächenelektroden an der Herzaußenseite angelegt werden, um die für eine Defibrillation erforderliche hohe Energie übertragen zu können. Die Wahrnehmung einer Herzrhythmusstörung erfolgt dabei durch die Elektrode in der rechten Herzkammer, wobei eine elektrische Potentialdifferenz zwischen zwei etwa einen halben bis einen Zentimeter voneinander getrennten Punkten in der Herzspitze abgeleitet oder gemessen wird. Eine solche Potentialdifferenz entsteht bei jedem Herzschlag durch die diesem vorausgehende elektrische Erregungsausbreitung am Herzen. Wenn bestimmte Interventionskriterien erreicht werden, gibt die Steuerungseinheit des Defibrillators das entsprechende Behandlungsprogramm frei und beseitigt die Herzrhythmusstörung durch die gezielte Abgabe bestimmter Impulse. In der Mehrzahl der Fälle ist dabei die Abgabe eines Elektroschocks mit zwei bis vierzig Joule erforderlich. Dies führt in bekannter Weise zu einer elektrischen Gleichrichtung aller Herzmuskelzellen und damit zur Wiederherstellung des normalen Herzschlages.

Da solche implantierten Systeme nur eine begrenzte Energiekapazität in der mit eingebauten Batterie haben, kann nur eine begrenzte Anzahl solcher Defibrillationsschocks abgegeben werden, wonach ein, operativer Austausch notwendig wird. Je seltener es also zu Herzrhythmusstörungen kommt, um so länger kann das jeweilige Gerät im Patienten verbleiben und um so geringer wird die Gefahr für den Patienten selbst, daß eine einmal aufgetretene lebesbedrohliche Herzrhythmusstörung sich nicht mehr beheben läßt.

EP-A-554 208 offenbart eine Vorrichtung zur Analyse von Mangeldurchblutung. Die Elektroden können jedoch nicht paarweise beliebig zusammengeschaltet werden und sind nicht geeignet im Herzen plaziert zu werden. Das EP-Dokument zeigt somit die Merkmale in den ersten vier Zeilen des Anspruchs 1. Dieses EP-Dokument fällt unter Artikel 54(3) EPÜ.

Der Erfindung liegt deshalb die Aufgabe zugrunde, eine Vorrichtung der eingangs erwähnten Art zu schaffen, mit welcher Potentialdifferenzen von elektrischen Signalen des Herzens erfaßt und darauf hin ausgewertet werden können, ob eine Mangeldurchblutung des Herzens vorliegt und eine Herzrhythmusstörung zu erwarten ist.

Diese Aufgabe wird erfindungsgemäß dadurch gelöst, daß die Vorrichtung gemäß Anspruch 1 gestaltet ist.

Die Erfindung macht sich also die Erkenntnis zunutze, daß lebensbedrohlichen Herzrhythmusstörungen häufig eine auslösende Mangeldurchblutung vorausgeht und diese an dem Potentialverlauf der Herzerregung und zwar aufgrund von Veränderungen innerhalb des ST-Segmentes bzw. der T-Welle erkennbar ist. Tritt eine solche Veränderung im Potentialverlauf auf, können also entsprechende Gegenmaßnahmen veranlaßt und dadurch zumindest in einer großen Zahl von Fällen das Auftreten der eigentlichen gefährlichen Herzrhythmusstörung unterbunden werden.

Weil beim Messen zwei Pole zusammengeschaltet sind und das elektrische Potential dieser beiden Pole gegenüber dem dritten Pol gemessen wird, ergibt sich eine deutliche Ableitung des Potentialverlaufes oder des Erregungsablaufes am Herzen.

Dabei können. zumindest die im Herzen implantierbaren Pole zusammengeschaltet sein und es kann das elektrische Potential gegenüber dem außenliegenden Pol meßbar sein. Auf diese Weise kann ein großer Teil der Herzmasse erfaßt werden, so daß mit großer Sicherheit Veränderungen des Potentialverlaufes rechtzeitig erkannt werden können.

Um noch größere Teile des Herzens und Mangeldurchblutungen an möglichst allen Stellen des Herzens erkennen zu können, ist die Auswerte- und Steuereinrichtung zum wahlweisen Zusammenschalten von zwei der drei Elektroden ausgebildet. Es kann dann also jeweils gegenüber dem verbleibenden Pol das elektrische Potential gemessen werden, so daß sich drei Ableitungskonfigurationen ergeben können, die jeweils entsprechende Herzbereiche umfassen und so eine Lokalisierung einer Mangeldurchblutung erlauben.

Besonders günstig ist es dabei, wenn die Polzuordnung mit wechselndem Zusammenschalten jeweils zweier Pole rotierend insbesondere jeweils nach einem oder mehreren Herzschlägen veränderbar ist. Auf diese Weise kann praktisch permanent das gesamte Herz bezüglich einer Durchblutungsstörung überwacht und die eventuell auftretende Störung lakalisiert werden.

Wesentlicher Vorteil der Erfindung ist es dabei, daß eine ständige Überwachung eines Patienten möglich ist, der der Gefahr unterliegt, daß Herzrhythmusstörungen auftreten und diesen eine Mangeldurchblutung am Herzen vorausgeht. Gegenüber einer gelegentlichen Untersuchung mit Hilfe eine EKG ergibt sich also die Möglichkeit der ständigen Überwachung selbst dann, wenn ein Wechsel des Zusammenschaltens der jeweils zwei gegenüber dem dritten Pol zu messenden Elektroden nicht stattfindet. Das Zusammenschalten und Umschalten der Elektroden und ihrer Pole kann dabei über die implantierte, vorzugsweise batteriebetriebene Steuereinrichtung, insbesondere deren Mikroprozessor erfolgen. Somit kann ein gefährdeter Patient durch ein derartiges implantierbares System praktisch dauernd überwacht und im Falle einer Durchblutungsstörung rechtzeitig gewarnt werden.

Da ein sehr großer Anteil der Patienten, die ein implantierbares Defibrillationssystem erhalten, zeitweise Mangeldurchblutungen des Herzens haben, ist es besonders zweckmäßig, wenn die Elektroden mit einem implantierbaren Defibrillator und/oder Herzschrittmacher verbunden sind, der als Generatoreinheit ausgebildet und mit der Steuereinrichtung versehen ist. Die Vorrichtung kann also bei dieser Ausgestaltung besonders günstig angewendet werden, wenn ein Patient ein Defibrillationssystem oder einen Herzschrittmacher mit entsprechenden Elektroden benötigt, weil diese Teile und Geräte dann gleichzeitig dazu herangezogen werden können, den Patienten in der beschriebenen Weise zu überwachen.

Gemäß einem Ausführungsbeispiel kann die Auswerte- und Steuereinrichtung Teil des Herzschrittmachers und/oder des Defibrillators sein. Gerade solche Patienten, die einen Herzschrittmacher oder gar einen Defibrillator benötigen und diesen implantiert bekommen, sind besonders gefährdet, so daß in diesen Fällen das Implantieren einer zusätzlichen Elektrode besonders lohnend ist. Darüber hinaus können die zu implantierenden Geräte, also ein Herzschrittmacher und/oder ein Defibrillator, in ihrem Gehäuse die entsprechende Steuer- und Auswerteeinrichtung enthalten. Da eine verminderte Durchblutung rechtzeitig erkannt und behoben werden kann, muß dabei in vorteilhafter Weise ein starker Stromstoß für eine Defibrillation entsprechend seltener abgegeben werden.

Der außerhalb des Herzens befindliche Pol kann als Ring ausgebildet sein, der an der die beiden im Herzen implantierbaren Elektroden mit der Generatoreinheit verbindenden Leitung, diese umschließend, befestigt sein kann. Somit läßt sich dieser außerhalb des Herzens zu implantierende Pol günstig fixieren und plazieren.

Es ist aber auch möglich, daß der Pol der außerhalb des Herzens implantierbaren Elektrode das Gehäuse oder eine Verbindung mit dem Gehäuse der Generatoreinheit ist. Somit erhält dieses Gehäuse eine zusätzliche Funktion.

Das Generatorgehäuse, also das Gehäuse eines Herzschrittmachers oder eine Defibrillators, kann einen akustischen Signalgeber aufweisen oder enthalten, der durch eine Messung einer Mangeldurchblutung oder einer Herzrhythmusstörung auslösbar ist. Somit wird der Patient unmittelbar selbst bei einer Störung an seinem Herzen gewarnt, selbst wenn diese Störung nicht von ihm beispielsweise als Schmerz gefühlt wird. Er kann dann sofort entsprechende Gegenmaßnahmen ergreifen und dadurch eventuell weitere Schäden vermeiden.

Es ergibt sich also insgesamt, daß, die zur Verfügung stehendeden Ereignisdaten und die Registrierung der jeweils gespeicherten, abnormalen Werte oder ST-Streckenbeispiele zum Beispiel telemetrisch jederzeit abgefragt werden können und auch die akustischen Signale können dazu benutzt werden, den Patienten über den entsprechenden Befund zu informieren.

Eine besonders zweckmäßige Ausgestaltung der Erfindung kann dahin gehen und es ermöglichen, daß Abweichungen von dem gespeicherten Referenzwert als Steuerimpuls zum Applizieren eines Medikamentes und/oder eines elektrischen Impulses benutzt werden. Dazu kann die Steuereinrichtung mit einer implantierbaren Medikamentenpumpe gekoppelt sein und die, Pumpe kann durch eine Messung einer Mangeldurchblutung und/oder einer Herzrhythmusstörung einschaltbar sein. Somit kann die erfindungsgemäße Vorrichtung zusätzlich zu ihrer Diagnosefunktion auch eine Auslösefunktion für eine Therapie aufweisen. Ferner kann das Auftreten einer Durchblutungsstörung am Herzen auch als Steuerungskriterium oder -signal herangezogen werden, um entsprechende Gegenmaßnahmen mehr oder weniger automatisch ablaufen zu lassen.

Beispielsweise kann die Meßeinrichtung mit dem Herzschrittmacher oder Defibrillator derart gekoppelt sein, daß beim Auftreten von Herzrhythmusstörungen elektrische Impulse auf die Elektrodenpole geleitet werden. Somit kann vor allem bei dem Auftreten gefährlicher Herzrhythmusstörungen von dem ebenfalls implantierten Herzschrittmacher oder Defibrillator sofort automatisch die entsprechende Gegenmaßnahme durchgeführt werden. Die Vorrichtung erlaubt also sowohl das Erkennen von Mangeldurchblutungen als auch von Herzrhythmusstörungen und bei entsprechender Ausgestaltung auch gleich das Auslösen entsprechender Gegenmaßnahmen. Dabei genügt eventuell die Abgabe eines niederenergetischen Impulses, also eines Herzschrittmacherimpulses, mit einem definierten Ankopplungsinteryall oder einer vorgegebenen Herzfrequenz mit dem Ziel, diese Herzrhythmusstörung zu beseitigen. In schweren Fällen kann auch die Defibrillation ausgelöst werden. Die schon erwähnte automatische oder auch semi-automatische Medikamentenapplikation mit Hilfe eines implantierbaren Pumpsystemes kann dafür sorgen, daß entsprechende Medikamente beispielsweise in eine Vene eingeleitet werden.

Wie bereits erwähnt, kann die Vorrichtung einen Speicher zum Aufzeichnen der Meßergebnisse enthalten. Dies erlaubt es dem Arzt, beispielsweise durch telemetrisches Abrufen der Meßergebnisse, einen entsprechenden Potentialverlauf über eine längere Zeit nachträglich zu kontrollieren und daraus Schlüsse über den Zustand des Herzens des Patienten zu ziehen und eventuelle weitere Therapiemaßnahmen zu ergreifen.

Um eventuell Energie zu sparen und .den Meßverlauf auf bestimmte Tageszeiten oder Ereignisse zu konzentrieren, kann die Vorrichtung einen Zeitgeber enthalten, der in einem bestimmten, vorgewählten Zeitrhythmus Messungen an den Elektroden auslöst.

Eine Schaltung der erfindungsgemäßen Vorrichtung kann vorsehen, daß die Meßeinrichtung mit den Elektroden verbundene Multiplexer zum Umschalten der einzelnen Elektroden jeweils wahlweise auf zwei Summierverstärker aufweist, deren Ausgänge an einen Analog-Digital-Konverter angeschlossen sind und der Analog-Digital-Konverter kann mit dem Mikroprozessor verbunden sein. Dies ergibt eine einfache Schaltung, um jeweils zwei Elektroden zusammenzuschalten und das Potential zwischen diesen und der dritten Elektrode zu messen und zu speichern.

Insgesamt ergibt sich ein Verfahren und auch eine Vorrichtung, womit eine Myokardischämie nachgewiesen werden kann, die morphologische Änderungen im Bereich der Erregungsrückbildung eines erfaßten Signales bei jedem einzelnen Herzschlag bewirkt. Trägt man den elektrischen Potentialverlauf über die Zeit auf, erhält man einen sogenannten QRS-Komplex, welcher der Ausbreitung der elektrischen Energie von der Herzbasis zur Herzspitze entspricht. Diese, elektrische Erregung ist Voraussetzung für die nachfolgende mechanische Kontraktion. Die erfaßten elektrischen Potentiale liegen im Bereich von 10 bis 100 Hertz. Nach dem QRS-Komplex folgt die schon erwähnte ST-Strecke, die die Repolarisation des Herzens erfaßt und ein Signal mit niedriger Amplitude von zum Beispiel 0,05 bis 20 Hertz darstellt. Insbesondere Anomalien in diesem Bereich des Potentialverlaufes können durch die erfindungsgemäße Vorrichtung erfaßt und als Warnsignal verwendet werden, denn eine Mangeldurchblutung oder Myokardischämie ist an einer morphologischen Änderung des elektrischen Signales in dieser Erregungsrückbildungsphase der ST-Strecke erkennbar. Dabei können folgende morphologischen Abweichungen im Vergleich zu einem Referenzsignal eines normalen Verlaufes berücksichtigt werden:
- Formänderung der T-welle
- T-Wellenanstiegssteilheit
- Flächenänderung und der T-Welle
- Dauer der T-Welle
- ST-Strechendauer
- Absenkung oder Anhebung des J-Punktes.

Es ergibt sich also die Möglichkeit, eine Myokardischämie rechtzeitig zu erkennen, um Maßnahmen zu ergreifen, die eine davon ausgelöste Herzrhythmusstörung vermeiden.

In vorteilhafter Weise kann die Erfindung jedoch auch dazu ausgenutzt werden, Herzrhythmusstörungen selbst danach zu differenzieren, ob sie von den Herzvorhöfen oder den Herzkammern ausgehen. In Abhängigkeit vom Ergebnis einer entsprechenden Signalanalyse kann nach Erkennung von Herzrythmusstörungen ausgehend von den Herzvorhöfen oder Herzkammern ein entsprechendes automatisches Interventionsprogramm gestartet werden. Auch dabei kann die Signalanalyse jeweils als Steuerungssignal für eine solche Intervention dienen. Diese kann wiederum in Form einer Medikamentenapplikation oder auch eines elektrotherapeutischen Vorgehens mit Hilfe des implantierten Systemes erfolgen.

Nachstehend sind Ausführungsbeispiele der Erfindung anhand der Zeichnung näher beschrieben. Es zeigt in schematisierter Darstellung:
- Fig.1: ein Herz mit zwei von einem Defibrillator ausgehenden Elektroden, deren Pole in der Herzkammer und im Herzvorhof angeordnet sind, wobei eine dritte ringförmig um die Zuleitung gelegte Elektrode außerhalb des Herzens vorgesehen ist,
- Fig.2: eine abgewandelte Ausführungsform, bei welcher zwei Elektroden mit ihren Polen im Herzen plaziert sind und das Gehäuse einer Steuereinheit oder eines Herzschrittmachers einen dritten, außerhalb des Herzens befindlichen Pol bildet,
- Fig. 3: eine Anordnung analog Fig.2, wobei zusätzlich eine Flächenelektrode an der Außenseite des Herzens vorgesehen ist,
- Fig. 4: eine Anordnung mit einem implantierten Herzschrittmacher und davon ausgehen, den, im Herzen implantierten Elektroden sowie einer zusätzlichen, außerhalb des Herzens implantierten Elektrode,
- Fig. 5: ein Schaltbild, bei welchem jeweils zwei der implantierten Elektroden zusammengeschaltet und gegenüber einer dritten Elektrode das Potential gemessen wird,
- Fig.6a bis Fig.6e: jeweils den Verlauf einer Herzerregung bei einem Herzschlag, wobei Fig.6a einen normalen Verlauf, die Figuren 6b bis Fig. 6d einen durch Mangeldurchblutung veränderten Verlauf vor allem im Bereich der Erregungsrückbildung und Fig.6e wiederum einen normalisierten Verlauf zeigen,
- Fig.7: den Potentialverlauf über die Zeit im Falle einer Herzrhythmusstörung,
- Fig.8: in schematisierter Blockdarstellung den Ablauf beim Erkennen einerseits einer Durchblutungsstörung und andererseits einer Herzrhythmusstörung mit Andeutung von daraus abgeleiteten Steuerfunktionen,
- Fig.9: den Potentialverlauf an einem Herzen, bei welchem einer Herzrhythmusstörung eine Durchblutungestörung vorausgeht, und
- Fig.10: in vergrößertem Maßstab ein weiteres Beispiel für den Potentialverlauf über die Zeit, bei welchem zunächst eine Durchblutungsstörung und dann eine Herzrhythmusstörung auftreten.

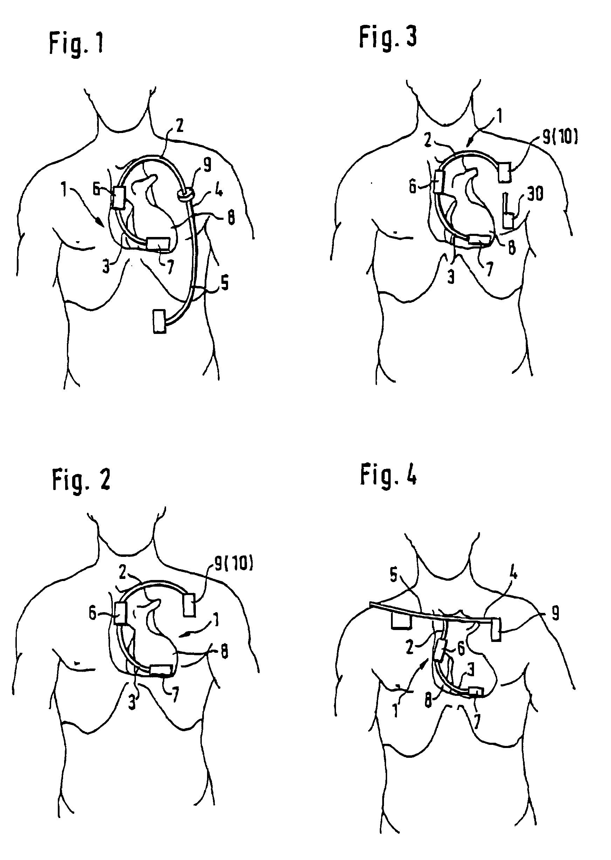

Eine in den Figuren 1 bis 4 jeweils schematisch angedeutete Vorrichtung 1 dient neben einer anderen noch zu beschreibenden Funktion dazu, Mangeldurchblutungen am Herzen oder Herzrhythmusstörungen wahrzunehmen oder zu erfassen. Die Vorrichtung 1 weist dazu insgesamt drei implantierte Elektroden 2,3 und 4 auf, die dabei teilweise ineinandergeführt sind, so daß eine gemeinsame Leitung 5 mit entsprechend mehreren Adern oder Elektroden vorhanden ist.

Zwei Elektroden 2 und 3 sind mit ihren Polen 6 und 7 im Herzen 8 implantiert, während die dritte Elektrode 4 mit ihrem Pol 9 außerhalb des Herzens 8 neben diesem implantiert ist, wobei die Anordnung bei allen Ausführungsbeispielen so getroffen ist, daß die Verbindungslinien der drei Pole 6,7 und 9 ein zumindest einen Teil des Herzens 8 - und zwar einen möglichst großen Teil des Herzens 8 - als Dreieck umschließen oder umgreifen.

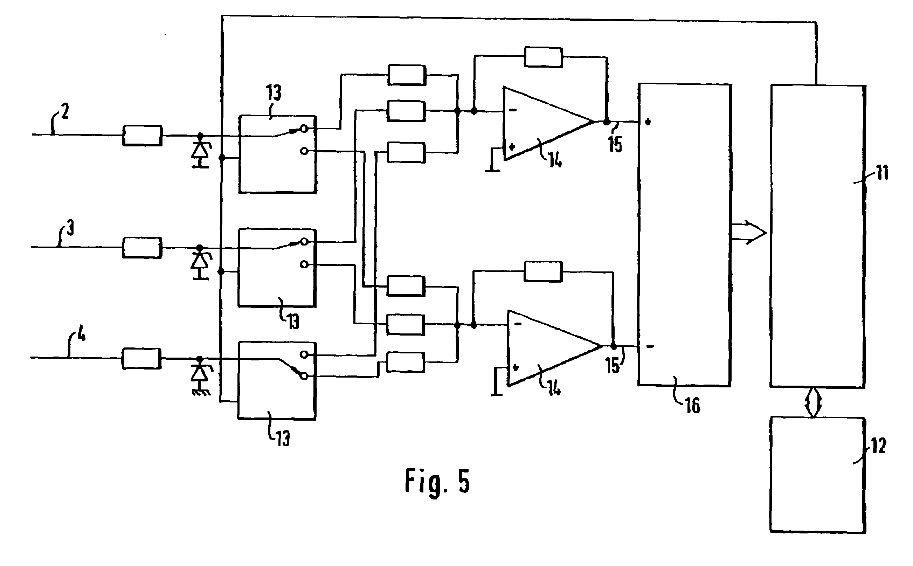

Die Elektroden 2, 3 und 4 sind dabei über die Leitung 5 mit einer ebenfalls implantierten Auswerte- und Steuereinrichtung 10 verbunden. Ein schematisches Schaltbild dieser Auswerte- und Steuereinrichtung 10 ist insgesamt in Fig.5 dargestellt.

Vor allem in Fig.5 erkennt man, daß die Auswerte- und Steuereinrichtung 10 einen Mikroprozessor 11 zur Steuerung sowie einen damit verbundenen Speicher 12 zur Speicherung von insbesondere als Referenzwerte dienenden Meßdaten enthält, wobei der Speicher 12 aber auch durch laufende Messungen erzeugte Daten speichern und abrufbar zur Verfügung stellen kann.

Da die Steuer- und Auswerteeinrichtung 10 implantiert ist und Messungen von Mangeldurchblutungen oder von Herzrhythmusstörungen vor allem bei Personen mit geschädigtem Herzen erforderlich oder sinnvoll sind, ist im Ausführungsbeispiel die Auswerte- und Steuereinrichtung 10 Teil eines implantierten Herzschrittmachers (Fig.4) oder eines Defibrillators (Fig.1). Somit können eventuell festgestellte Störungen gleich zur Steuerung dieser zur Elektrotherapie dienenden implantierten Geräte in noch zu beschreibender Weise herangezogen werden. Gegenüber solchen Patienten, denen ein Herzschrittmacher oder ein Defibrillator implantiert ist, ergibt sich also lediglich das Implantieren einer zusätzlichen, außerhalb des Herzens zu plazierenden Elektrode 4 mit einem Pol 9.

Gemäß Fig.1 kann dieser außerhalb des Herzens 8 befindliche Pol 9 als Ring ausgebildet sein, der an der die beiden im Herzen 8 implantierten Elektroden 2 und 3 mit der Generatoreinheit bzw. Auswerteeinrichtung 10 verbindenden Leitung 5, diese umschließend, befestigt ist. Somit wird dieser Pol 9 im Körper so fixiert, daß er seine gewünschte Lage trotz Körperbewegungen beibehält.

Bei den Ausführungsformen gemäß Fig.2 und 3 ist der Pol 9 der außerhalb des Herzens 8 implantierten Elektrode 4 das Gehäuse oder eventuell eine Verbindung mit dem Gehäuse der Generatoreinheit bzw. Auswerteeinrichtung 10.

Gemäß Fig.5 ist die Auswerte- und Steuereinrichtung 10 zum wahlweisen Zusammenschalten von zwei der drei Elektroden und zur Messung jeweils des Potentiales zwischen den zusammengeschalteten Elektroden einerseits und der dritten Elektrode andererseits bzw. deren Polen ausgebildet. Man erkennt in Fig.5, daß die Meßeinrichtung 10 mit den Elektroden 2, 3 und 4 verbundene Multiplexer 13 zum Umschalten der einzelnen Elektroden jeweils wahlweise auf zwei Summierverstärker 14 aufweist, deren Ausgänge 15 an einen Analog-Digital-Converter 16 angeschlossen sind. Der Analog-Digital-Converter 16 ist wiederum mit dem Mikroprozessor 11 verbunden, der die Multiplexer 13 ansteuert. Somit kann gemäß einem in dem Mikroprozessor 11 eingegebenen Programm ein wechselweises Zusammenschalten jeweils zweier Elektroden und Messen gegenüber der dritten Elektrode stattfinden, so daß an dem Herzen 8 jeweils unterschiedliche Stellen des Herzens 8 erfaßt und dort eventuell auftretende Störungen erkannt werden können.

Gegebenenfalls kann das Generatorgehäuse, das heißt das Gehäuse der Meß- und Auswerteeinrichtung 10 mit Herzschrittmacher oder Defibrillator einen akustischen Signalgeber aufweisen, der durch eine Messung einer Mangeldurchblutung oder einer Herzrhythmusstörung auslösbar ist. Wird also durch den Meßvorgang eine gegenüber gespeicherten Referenzwerten abweichender Potentialverlauf erfaßt oder gemessen, kann der Patient sofort durch ein akustisches Signal gewarnt werden, um entsprechende Gegenmaßnahmen wie Aufgabe anstrengender Tätigkeiten, Einnahme von Medikamenten oder Aufsuchen eines Arztes zu ergreifen.

Nicht dargestellt ist die Möglichkeit, die Steuereinrichtung 10 mit einer ebenfalls implantierten Medikamentenpumpe zu koppeln und diese Pumpe durch eine Feststellung einer Mangeldurchblutung und/oder einer Herzrhythmusstörung einschaltbar oder ansteuerbar zu machen, so daß automatisch in einem solchen akuten Fall das erforderliche Medikament gegeben wird.

Der Speicher 12 kann - wie schon erwähnt - zum Aufzeichnen der Meßergebnisse dienen, so daß ein Arzt von Zeit zu Zeit den Potentialverlauf am Herzen zum Beispiel telemetrisch abrufen kann, um auch daraus Rückschlüsse über erforderliche Therapiemaßnahmen oder auf den jeweiligen Zustand des Herzens zu ermöglichen.

Die Vorrichtung 1 kann auch noch einen Zeitgeber enthalten, der in einem bestimmten vorgewählten Zeitrhythmus Messungen an den Elektroden 2, 3 und 4 und deren Polen 6, 7 und 9 auslöst, so daß nicht unbedingt eine Dauerüberwachung, wohl aber eine Überwachung zu besonders kritischen Zeiten vorgenommen werden kann.

In zweckmäßiger Weise kann die Meßeinrichtung 10 mit dem Herzschrittmacher oder Defibrillator derart gekoppelt sein, daß beim Auftreten von Herzrhythmusstörungen elektrische Impulse auf die Elektrodenpole geleitet werden, die im Herzen implantiert sind. Somit können eventuelle Meßergebnisse sofort zu Gegenmaßnahmen benutzt werden.

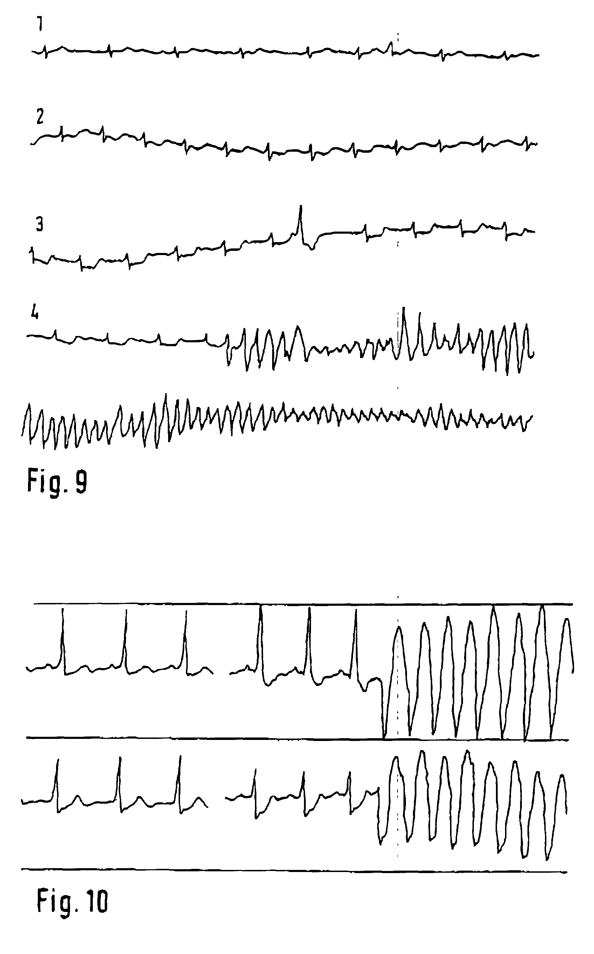

Eine Mangeldurchblutung der das Herz versorgenden Gefäße, als des Herzens, führt zu einer Instabilität der elektrischen Erregungsausbreitung am Herzmuskel und kann gemäß Fig.7, 9 und 10 auch eine Herzrhythmusstörung zur Folge haben. Herzrhythmusstörungen können durch elektrische Impulse zwar beseitigt werden, jedoch kommt es darauf an, diese rechtzeitig zu erkennen. Wenn es gelingt, die mangelnde Durchblutung rechtzeitig festzustellen, kann also eine Früherkennung durch eine permanente Überwachung eines Patienten erreicht werden, so daß entsprechende Gegenmaßnahmen rechtzeitig möglich werden.

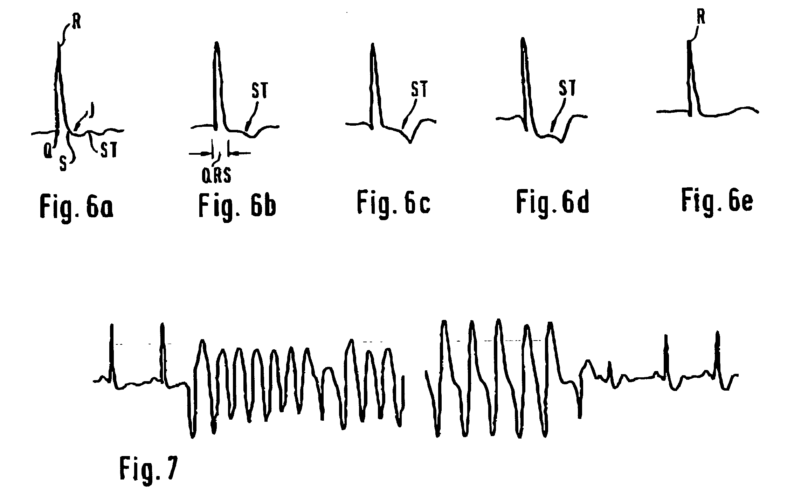

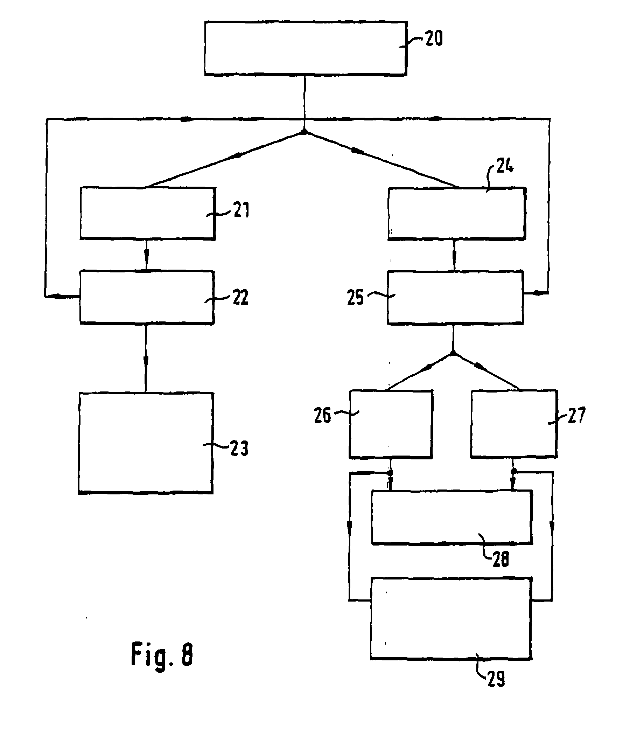

Trägt man über die Zeit den elektrischen Potentialverlauf am Herzen auf, entstehen jeweils Kurven etwa gemäß Fig.6a, die aneinandergereiht die Funktion des Herzens bzw. der Herzschläge darstellen und von EKG-Messungen auch bekannt sind. Diese elektrische, in diesen Kurven dargestellte Erregung ist Voraussetzung für die nachfolgende mechanische Kontraktion des Herzens 8. Dabei ist ein sogenannter QRS-Komplex als deutliche und charakteristische Spitze im Kurvenverlauf erkennbar, woran sich hinter dem sogenannten J-Punkt die ST-Strecke anschließt, die die Repolarisation des Herzens umfaßt. In den Fig. 6b bis 6d erkennt man, wie diese ST-Strecke unter Umständen von dem in Fig.6a erkennbaren Normalverlauf abweichen kann, wenn das Herz in den durch die Messung des Potentiales erfaßten lokalen Bereich mangelhaft durchblutet ist. Die Ausschlagrichtung der elektrischen Erregung über die Zeit ist dabei abhängig von der Lage und Kombination des durch die Pole 6, 7 und 9 gebildeten Ableitungsdreieckes im Raum, ist dann aber für jeden Herzschlag identisch.

Tritt in der EKG-Kurve eine morphologische Änderung des elektrischen Signales in der Erregungsrückbildungsphase, also der ST-Strecke der Kurve auf, wie es in den Figuren 6b und in zunehmendem Maße in Fig.6c und schließlich 6d erkennbar ist, ist dies ein Hinweis auf eine Mangeldurchblutung. Durch Vergleich mit einem abgespeicherten Referenzwert etwa gemäß Fig.6a kann dieser Zustand einer Durchblutungsstörung erfaßt und angezeigt oder zur automatischen Einleitung von Therapiemaßnahmen ausgenutzt werden. In den Figuren 7, 9 und 10 sind jeweils Beispiele dargestellt, wie einer Herzrhythmusstörung mit starken Ausschlägen und völliger Abweichung von dem üblichen Potentialverlauf solche Potentialverläufe mit geänderten ST-Strecken vorausgehen. Dabei können folgende morphologischen Abweichungen im Vergleich zum Referenzsignal berücksichtigt werden:
- Formänderung der T-Welle,
- T-Wellenanstiegssteilheit,
- Flächenänderung unter der T-Welle,
- Dauer der T-Welle,
- ST-Streckendauer,
- Absenkung oder Anhebung des J-Punktes.

Wie schon erwähnt, zeigt Fig.6a ein normales Signal, welches bei ständiger Wiederholung auf gesunde Verhältnisse schließen läßt. Während einer Mangeldurchblutung des Herzens kommt es jedoch gemäß den Figuren 6b, 6c und 6d zu ischämischen Veränderungen der Erregungsrückbildung, was der Vergleich der Figuren deutlich zeigt. Fig.6e zeigt dann wieder den Zustand nach einer Normalisierung, wenn die ausreichende Durchblutung des Herzmuskels wieder hergestellt ist. Die jeweilige Signalanalyse mit Hilfe der Steuer- und Auswerteeinrichtung 10 kann kontinuierlich oder in vorgegebenen Zeitintervallen - mit Hilfe des erwähnten Zeitschaltgliedes - oder auch oberhalb bestimmter Herzfrequenzen erfolgen. Auch kann das Analyseintervall bei bestimmten Ereignissen verkürzt oder verlängert werden.

Sofern eine Mangeldurchblutung im Vergleich zum Referenzsignal auftritt, können die entsprechenden abnormalen ST-Strecken in digitaler Form oder als numerisches Ereignis in dem Speicher 12 abgespeichert werden, um für eine anschließende telemetrische Abfrage jederzeit zur Verfügung zu stehen. Wie erwähnt, können jedoch auch akustische Signale abgegeben werden, um den Patienten selbst zu Gegenmaßnahmen zu veranlassen. Die schon erwähnt Applikation eines Medikamentes über ein gekoppeltes Pumpinfusionssystem kann dabei eventuell über eine im Bereich der Elektroden liegenden Zugang zum Herzen erfolgen.

In Fig.8 ist ein Beispiel eines solchen Erkennungsablaufes mit entsprechender Steuerungsfunktion dargestellt:
Die schon erwähnte Speicherung von Referenzdaten ist dabei in Fig. 8 mit 20 bezeichnet. Davon ausgehend kann beispielsweise bei einer Herzfrequenz von mehr als 100 alle drei Minuten eine ST-Signalanalyse 21 erfolgen. Ergibt diese eine Mangeldurchblutung 22, kann der dabei festgestellte Wert der Speicherung 20 zugeführt werden. Außerdem kann in dem mit 23 bezeichneten Schritt ein akustisches Signal gegeben werden, eine Medikamentenpumpe in Gang gesetzt werden oder eine Stimulation bewirkt werden.

Parallel dazu kann auch durch die Elektroden eine Messung oder Kontrolle bei jedem Herzschlag erfolgen, ob eine Herzrhythmusstörung vorliegt, welcher Schritt in dem Schema gemäß Fig. 8 mit 24 bezeichnet ist. Ergibt sich ein Normalbefund - bei 25 - kann dieser ebenfalls abgespeichert werden.

Erfolgt jedoch kein Normalbefund, kann die Messung zeigen, ob die Arrhythmie vom Vorhof oder von der Herzkammer ausgeht, was in dem Schema mit 26 und 27 gekennzeichnet ist. Die daraus abgeleiteten Werte können zur Speicherung 28 herangezogen werden und außerdem wiederum bei einem Schritt 29 dazu dienen, ein akustisches Signal abzugeben, eine Medikamentenpumpe in Gang zu setzen, eine Stimulation des Herzens durchzuführen oder eine Defibrillation zu veranlassen.

Das von jeder der drei möglichen Ableitung Konfigurationen zur Verfügung stehende Signal der elektrischen Erregungsausbreitung und -rückbildung eines Herzschlages kann also gemäß dem rechten Ast des Schemas in Fig.8 auch zur Differenzierung von Herzrhythmusstörungen dienen, die von den Herzvorhöfen oder den Herzkammern ausgehen. Dazu wird das elektrische Signal herangezogen, welches bei der Erregungsausbildung entsteht, also der QRS-Komplex mit der deutlichen und im Normalfall relativ hohen Spitze. Eine normale Erregungsausbreitung oder Rhythmusstörungen, die in den Vorhöfen entstehen, sind durch ein schnelles Signal von weniger als 100 msec bei der Erregung der Herzkammern gekennzeichnet und die Ausbreitungsrichtung im Raum verläuft von der Herzbasis zur Herzspitze. Herzrhythmusstörungen, die in den Herzkammern entstehen, sind durch morphologische Änderungen des elektrischen Signales gekennzeichnet, unter anderem durch Verbreiterung des QRS-Komplexes, Formänderung, Änderung der Fläche unter der elektrischen QRS-Kurve und Änderung der Flankensteilheit des Signales.

Wie bei der Signalanalyse zur Erfassung einer Durchblutungsstörung wird auch zur Erfassung solcher Rhythmusstörungen zunächst gemäß dem Schritt 20 ein. Referenzsignal abgespeichert, welches in seiner Morphologie dann mit den Signalen der neuen Herzschläge verglichen werden kann. Eine Abweichung um einen bestimmten, vorgegebenen Betrag qualifiziert den Ursprung der elektrischen Herzerregung ausgehend von den Herzvorhöfen oder den Herzkammern. Bei dieser Bewertung können in vorteilhafter Weise zusätzliche Informationen wie Herzfrequenz oder ein einständig erfaßtes Signal der Vorhoferregung über die entsprechende Elektrode herangezogen werden.

Die Basis zur Erfassung des Ausbreitungssignales der elektrischen Erregung eines Herzschlages wird durch die erfindungsgemäße tripolare Intra-Extrakardiale Ableitungskonfiguration gebildet. Auch dabei werden jeweils die Potentiale zweier Elektroden gegenüber einer dritten Elektrode gemessen. Zur Differenzierung von Herzrhythmusstörungen, die von den Herzvorhöfen oder den Herzkammern ausgehen, kann allerdings bereits die Ableitung oder Messung des elektrischen Signals zwischen nur zwei der drei zur Verfügung stehenden Ableitungsmöglichkeiten ausreichen.

Sofern Abweichungen des elektrischen Signales gegeben sind, wird der jeweilige QRS-Komplex oder das Ereignis als solches im Mikroprozessor bzw. Speicher abgespeichert. Ferner kann es dem Patienten zum Beispiel akustisch übermittelt oder zu jedem Zeitpunkt telemetrisch durch den Arzt abgerufen werden.

In Abhängigkeit vom Ergebnis der Signalanalyse kann nach Erkennung von Herzrhythmusstörungen ausgehend von den Herzvorhöfen oder Herzkammern ein entsprechendes automatisierts Interventionsprogramm gestartet werden. Dabei dient die Signalanalyse jeweils als Steuerungssignal für eine solche Interventiön, die zum Beispiel in Form einer automatischen oder semiautomatischen Medikamentenapplikation über ein implantiertes Pumpsystem erfolgen kann, wobei ein Medikament in eine Vene eingepumpt und eingeleitet wird. Die Erkennung von Herzrhythmusstörungen kann ferner dazu benutzt werden, elektrotherapeutische Therapiemaßnahmen zu veranlassen. So können Niederenergetische Impulse von einem Herzschrittmacher mit einem definierten Ankopplungsintervall oder einer vorgegebenen Herzfrequenz abgegeben werden mit dem Ziel, die Herzrhythmusstörung zu beseitigen.

Bei schweren Herzrhythmusstörungen, die bei der Signalanalyse durch entsprechend vorgegebene Kriterien identifiziert werden, kann die unmittelbare Abgabe eines Elektroschocks zur Beendigung der Herzrhythmusstörung erforderlich sein und durch den Defibrillator ausgelöst werden. Die morphologische Signalanalyse wird also auch hier zur Steuerung der Therapiemaßnahme eingesetzt.
Das Verfahren zur Signalanalyse des elektrischen Potentialverlaufes der Herzerregung zum Beispiel bei Mangeldurchblutung am Herzen und/oder bei einer Herzrhythmusstörung sieht vor, daß der Potentialverlauf mittels einer Elektrodenanordnung mit wenigstens drei Elektroden 2, 3 und 4 gemessen wird, daß wenigstens zwei der drei Elektroden im Herzen 8 implantiert werden, wobei ein Pol 7 der einen Elektrode 3 in der rechten Herzkammer und ein Pol 6 der anderen Elektrode 2 im rechten Vorhof oder in der oberen Hohlvene plaziert werden. Eine dritte Elektrode 4 wird mit ihrem Pol 9 außerhalb des Herzens 8 so implantiert, daß die Verbindungslinien der drei Pole ein einen Teil des Herzens 8 umgreifendes Dreieck bilden. Jeweils die Potentialdifferenz zwischen einer der Elektroden und den beiden zusammengeschalteten Elektroden kann gemessen und der Signalverlauf ausgewertet oder einer Auswerteeinrichtung zugeführt werden. Die Auswertung kann zum Einleiten entsprechender Therapiemaßnahmen verwendet werden.
In Fig. 3 ist noch eine Flächenelektrode 30 angedeutet, mit welcher an der Herzaußenseite ein Defibrillations-Stromstoß appliziert werden kann.

## Patentansprüche

1. Vorrichtung zur Signalanalyse bei einer Mangeldurchblutung am Herzen mittels Meßelektroden zum Erfassen des elektrischen Potentialverlaufes der Herzerregung über die Zeit mit wenigstens drei implantierbaren Elektroden (2,3,4), wobei zwei der Elektroden in Gebrauchsstellung mit ihren Polen (6,7) im Herzen (8) und die dritte Elektrode mit ihrem Pol (9) außerhalb des Herzens (8) derart plaziert werden können, daß die Verbindungslinien der drei Pole (6,7,9) ein einen Teil des Herzens (8) umgreifendes Dreieck bilden, wobei ein Speicher (12) zum Erfassen und Speichern des elektrischen Potentialverlaufes eines normalen Herzschlages über die Zeit als Referenzwert vorgesehen ist und die späteren Messungen mit dem abgespeicherten Referenzwert vergleichbar sind, daß die Elektroden (2, 3, 4) mit einer ebenfalls implantierbaren Auswerte- und Steuereinrichtung (10) zur Messung jeweils des Potentiales zwischen zwei zusammengeschalteten Elektroden einerseits und der dritten Elektrode andererseits verbunden sind, wobei jeweils die Potentialdifferenz zwischen einer der Elektroden und den beiden anderen Elektroden meßbar und der Signalverlauf der Auswerte- und Steuereinrichtung (10) zuführbar ist, und daß die Auswerte- und Steuereinrichtung (10) zum derartigen wahlweisen Zusammenschalten von zwei der drei Elektroden ausgebildet ist, daß sich drei Ableitungskonfigurationen ergeben können.

2. Vorrichtung nach Anspruch 1, **dadurch gekennzeichnet, daß** die Auswerte,- und Steuereinrichtung (10) einen Mikroprozessor (11) zur Steuerung sowie den damit verbundenen Speicher (12) zur Speicherung von als Referenzwerten dienenden Meßdaten enthält.

3. Vorrichtung nach Anspruch 1, **dadurch gekennzeichnet, daß** die Polzuordnung mit wechselndem Zusammenschalten jeweils zweier Pole rotierend insbesondere jeweils nach einem oder mehreren Herzschlägen veränderbar ist.

4. Vorrichtung nach einem der Ansprüche 1 bis 3, **dadurch gekennzeichnet, daß** die Elektroden (2,3,4; 30) mit einem implantierbaren Defibrillator und/oder Herzschrittmacher verbunden sind, der als Generatoreinheit ausgebildet und mit der Steuereinrichtung (10) versehen ist.

5. Vorrichtung nach Anspruch 4, **dadurch gekennzeichnet, daß** die Auswerte- und Steuereinrichtung (10) Teil des Herzschrittmachers und/oder des Defibrillators ist.

6. Vorrichtung nach Anspruch 4, **dadurch gekennzeichnet, daß** der Pol (9) der außerhalb des Herzens (8) implantierbaren Elektrode (4) das Gehäuse oder eine Verbindung mit dem Gehäuse der Generatoreinheit ist.

7. Vorrichtung nach Anspruch 6, **dadurch gekennzeichnet, daß** das Generatorgehäuse einen akustischen Signalgeber aufweist oder enthält, der durch eine Messung einer Mangeldurchblutung oder einer Herzrhythmusstörung auslösbar ist.

8. Vorrichtung nach einem der Ansprüche 1 bis 7, **dadurch gekennzeichnet, daß** die Steuereinrichtung (10) mit einer Medikamentenpumpe gekoppelt ist und die Pumpe durch eine Messung einer Mangeldurchblutung und/oder einer Herzrhythmusstörung einschaltbar ist.

9. Vorrichtung nach einem der Ansprüche 1 bis 8, **dadurch gekennzeichnet, daß** sie einen Zeitgeber enthält, der in einem bestimmten, vorgewählten Zeitrhythmus Messungen an den Elektroden auslöst.

10. Vorrichtung nach Anspruch 4 oder 5, **dadurch gekennzeichnet, daß** die Auswerte- und Steuereinrichtung (10) mit dem Herzschrittmacher oder Defibrillator derart gekoppelt ist, daß beim Auftreten von Herzrhythmusstörungen elektrische Impulse auf die Elektrodenpole geleitet werden.

11. Vorrichtung nach einem der Ansprüche 2 bis 10, **dadurch gekennzeichnet, daß** die Auswerte- und Steuereinrichtung (10) mit den Elektroden verbundene Multiplexer (13) zum Umschalten der einzelnen Elektroden jeweils wahlweise auf zwei Summierverstärker (14) aufweist, deren Ausgänge (15) an einen Analog-Digital-Converter (16) angeschlossen sind, und daß der Analog-Digital-Converter (16) mit dem Mikroprozessor (11) verbunden ist.

## Claims

1. An apparatus for signal analysis in the event of a circulation deficiency in the heart by means of measurement electrodes to record the variation in electrical potential of cardiac excitation over time with at least three implantable electrodes (2, 3, 4), wherein in the position of use, two of the electrodes can be placed with their poles (6, 7) in the heart (8) and the third electrode can be placed with its pole (9) outside the heart (8) in such a way that the connecting lines of the three poles (6, 7, 9) form a triangle which surrounds a part of the heart, wherein a store (12) is provided to record and store the variation in electrical potential of a normal heartbeat over time as a reference value, and the later measurements can be compared with the stored reference value, that the electrodes (2, 3, 4) are connected with a likewise-implantable evaluation and control apparatus (10) for measuring the potential, in each case, between two connected electrodes on the one hand and the third electrode on the other hand, wherein in each case the potential difference between one of the electrodes and the two other electrodes can be measured and the signal variation can be supplied to the evaluation and control apparatus (10), and that the evaluation and control apparatus (10) is configured for such selective connection of two of the three electrodes so that three derivation configurations may result.

2. An apparatus according to claim 1, **characterised in that** the evaluation and control apparatus (10) contains a microprocessor (11) to effect control and the associated store (12) for storing measurement data which serves as reference values.

3. An apparatus according to claim 1, **characterised in that** the polar correlation, in each case with changeable connection of two rotating poles, can especially be altered in each case after one or more heartbeats.

4. An apparatus according to one of claims 1 to 3, **characterised in that** the electrodes (2, 3, 4; 30) are connected with an implantable defibrillator and/or cardiac pacemaker, which is configured as a generator unit and which is provided with the control apparatus (10).

5. An apparatus according to claim 4, **characterised in that** the evaluation and control apparatus (10) is a part of the cardiac pacemaker and/or the defibrillator.

6. An apparatus according to claim 4, **characterised in that** the pole (9) of the implantable electrode (4) outside the heart (8) is the housing or a connection with the housing of the generator unit.

7. An apparatus according to claim 6, **characterised in that** the generator housing has, or contains, an acoustic signal transmitter which can be triggered by measurement of a circulation deficiency or arrhythmia.

8. An apparatus according to one of claims 1 to 7, **characterised in that** the control apparatus (10) is coupled with a medicament pump and that the pump can be switched on by measurement of a circulation deficiency and/or arrhythmia.

9. An apparatus according to one of claims 1 to 8, **characterised in that** it contains a timer which triggers measurements at the electrodes in a specific, pre-determined time rhythm.

10. An apparatus according to claim 4 or 5, **characterised in that** the evaluation and control apparatus (10) is coupled with the cardiac pacemaker or defibrillator in such a way that electrical impulses are supplied to the electrode poles on occurrence of arrhythmia.

11. An apparatus according to one of claims 2 to 10, **characterised in that** the evaluation and control apparatus (10) has multiplexers (13) connected with the electrodes to switch the individual electrodes in each case selectively to two summing amplifiers (14) the outputs (15) of which are connected to an analogue-digital converter (16), and that the analogue-digital converter (16) is connected to the microprocessor (11).

## Revendications

1. Dispositif pour analyser les signaux lors d'une insuffisance de l'irrigation du coeur au moyen d'électrodes de mesure destinées à déterminer la courbe des potentiels électriques de la stimulation cardiaque en fonction du temps, avec au moins trois électrodes qui peuvent être implantées (2, 3, 4), deux des électrodes pouvant être placées dans le coeur (8) par leurs pôles (6, 7) en position d'utilisation, et la troisième pouvant être placée à l'extérieur du coeur (8) par son pôle (9), de telle manière que les droites passant par les trois pôles (6, 7, 9) forment un triangle qui entoure une partie du coeur (8), une mémoire (12) étant prévue pour déterminer et enregistrer la courbe du potentiel électrique d'un battement de coeur normal en fonction du temps, pour servir de valeur de référence, et que l'on peut comparer les mesures ultérieures à la valeur de référence qui a été mise en mémoire, par le fait que les électrodes (2, 3, 4) sont reliées à un dispositif d'exploitation et de commande (10) qui peut être également implanté et qui est destiné à mesurer à chaque fois le potentiel entre deux électrodes interconnectées, d'une part, et la troisième électrode, d'autre part, cependant que l'on peut mesurer à chaque fois la différence de potentiel entre l'une des électrodes et les deux autres électrodes et que l'on peut envoyer la courbe des signaux au dispositif d'exploitation et de commande (10) et par le fait que le dispositif d'exploitation et de commande (10) est conformé en vue de l'interconnexion au choix de deux des trois électrodes, de façon qu'il peut en résulter trois configurations de dérivées.

2. Dispositif selon la revendication 1, **caractérisé par le fait que** le dispositif d'exploitation et de commande (10) contient un microprocesseur (11) qui est destiné à la commande, ainsi que la mémoire (12) qui est reliée à celui-ci et qui est destinée à enregistrer des valeurs mesurées servant de valeurs de référence.

3. Dispositif selon la revendication 1, **caractérisé par le fait que** l'association des pôles par interconnexion alternée de deux pôles à chaque fois peut être modifiée d'une façon tournante, à chaque fois après un ou plusieurs battements du coeur.

4. Dispositif selon l'une des revendications 1 à 3, **caractérisé par le fait que** les électrodes (2, 3, 4 ; 30) sont reliées à un défibrillateur et/ ou à un stimulateur cardiaque qui peut être implanté, qui est réalisé sous la forme d'une unité génératrice et qui est pourvu du dispositif de commande (10).

5. Dispositif selon la revendication 4, **caractérisé par le fait que** le dispositif d'exploitation et de commande (10) fait partie du stimulateur cardiaque et/ ou du défibrillateur.

6. Dispositif selon la revendication 4, **caractérisé par le fait que** le pôle (9) de l'électrode (4) qui peut être implantée à l'extérieur du coeur (8) constitue le boîtier de l'unité génératrice ou une liaison avec ce boîtier.

7. Dispositif selon la revendication 6, **caractérisé par le fait que** le boîtier du générateur comporte ou contient un émetteur de signaux acoustiques qui peut être déclenché par la détection d'une insuffisance de l'irrigation du coeur ou d'une perturbation du rythme cardiaque.

8. Dispositif selon l'une des revendications 1 à 7, **caractérisé par le fait que** le dispositif de commande (10) est accouplé à une pompe à médicaments, et que la pompe peut être mise en route par la détection d'une insuffisance de l'irrigation du coeur ou d'une perturbation du rythme cardiaque.

9. Dispositif selon l'une des revendications 1 à 8, **caractérisé par le fait qu'**il contient une horloge qui déclenche des mesures sur les électrodes selon un rythme déterminé qui est choisi au préalable.

10. Dispositif selon la revendication 4 ou 5, **caractérisé par le fait que** le dispositif d'exploitation de commande (10) est accouplé au stimulateur cardiaque ou au défibrillateur d'une manière telle que des impulsions électriques soient envoyées aux pôles des électrodes lorsqu'il se produit des perturbations du rythme cardiaque.

11. Dispositif selon l'une des revendications 2 à 10, **caractérisé par le fait que** le dispositif d'exploitation et de commande (10) comprend des multiplexeurs (13) qui sont reliés aux électrodes pour commuter les électrodes individuelles à chaque fois sur deux amplificateurs totalisateurs (14) au choix, les sorties (15) de ceux-ci étant raccordées à un convertisseur analogique-numérique (16), et **par le fait que** le convertisseur analogique-numérique (16) est relié au microprocesseur (11).
